# EUROPÄISCHE PATENTSCHRIFT

(11) **EP 0 734 717 B1**
(45) Veröffentlichungstag und Bekanntmachung des Hinweises auf die Patenterteilung: **28.05.2003**
(21) Anmeldenummer: 96104390.8
(22) Anmeldetag: 20.03.1996
(51) Int. Cl.: A61K 7/11

(54) **Neue Haarfestigungsmittel**
New hair fixatives
Nouveaux fixateurs pour cheveux

(30) Priorität: 27.03.1995 DE 19510474
(43) Veröffentlichungstag der Anmeldung: 02.10.1996
(73) Patentinhaber: BASF AKTIENGESELLSCHAFT, 67056 Ludwigshafen (DE)
(72) Erfinder: Blankenburg, Rainer, Dr., 67067 Ludwigshafen (DE); Sperling, Karin, Dr., 67433 Neustadt (DE); Sanner, Axel, Dr., 67227 Frankenthal (DE)

(56) Entgegenhaltungen:
- EP-A- 0 219 830
- EP-A- 0 455 081
- EP-A- 0 635 257

## Beschreibung

Die vorliegende Erfindung betrifft neue Haarfestigungsmittel, die Polymermischungen als Filmbildner enthalten.

Haarsprayzusammensetzungen, die aus einem Polyester und einem wasserlöslichen Polymer, beispielsweise einem Copolymer aus N-Vinylcaprolactam und N-Vinylpyrrolidon bestehen, sind aus WO 93/17658 bekannt.

Solche Haarsprays sind jedoch hydrolyseempfindlich und daher in der Anwendung und Handhabung nur eingeschränkt verwendbar. Es bestand daher die Aufgabe, Haarfestigungsmittel zu entwickeln, die gute Festigereigenschaften bei guter Auswaschbarkeit und geringer Klebrigkeit aufweisen, ohne dabei die oben beschriebene Hydrolyseempfindlichkeit zu zeigen.

Demgemäß wurden Haarfestigungsmittel gefunden, enthaltend
A) 0,5 - 20 Gew.% eines Homo- oder Copolymeren aus mindestens 70 Gew.% N-Vinylcaprolactam (Polymer A), und
B) 0,5 - 20 Gew.% eines weiteren filmbildenden Polymeren (Polymer B), ausgewählt aus der Gruppe von Polyamiden, Polyurethanen, Homo- und Copolymeren von monoolefinisch ungesättigten Monomeren.

Als Polymere A sind Homopolymere des N-Vinylcaprolactam geeignet, die dem Fachmann bekannt sind und sich beispielsweise nach der in US 3 145 147 beschriebenen Vorschrift herstellen lassen.

Weitere geeignete Polymere A sind Copolymere aus N-Vinylcaprolactam und weiteren polymerisierbaren Monomeren, wobei die Copolymere aus mindestens 70 Gew.%, bevorzugt mindestens 85 Gew.% N-Vinylcaprolactam bestehen.

Als weitere polymerisierbare Monomere für die Copolymere A sind geeignet:

Monomere mit einer Säurefunktion wie Acrylsäure, Methacrylsäure, Acrylamido-methylpropylsulfonsäure (AMPS), (Meth)acrylsäure-3-sulfopropylester, gegebenenfalls auch in komplett oder teilweise neutralisierter Form;
C₁-C₁₈ Alkyl(meth)acrylate, wie tert. Butylacrylat, Ethylacrylat, iso-Butylmethacrylat, n-Butylmethacrylat, Methylmethacrylat, Ethylmethacrylat und Hydroxy-alkyl(meth)acrylsäureester; Vinylester der C₂-C₁₀ Fettsäuren wie Vinylacetat, Vinylpropionat und Vinylester längerkettiger und/oder verzweigter Fettsäuren, beispielsweise Versaticsäure;
C₃-C₈ N-Alkyl(meth)acrylamide, wie Methacrylamid, N,N-Dimethylacrylamid, N-tert. Butylacrylamid und N-tert. Octylacrylamid; N-Vinylpyrrolidon und N-Vinylpiperidon.

Die weiteren Monomeren können als einzelne Verbindung oder als Gemisch für das Copolymer A verwendet werden.

Solche Copolymere sind bekannt oder lassen sich durch üblichen Polymerisationsverfahren herstellen.

Beispielsweise werden in EP 455081 Copolymere aus N-Vinylpyrrolidon, N-Vinylcaprolactam und N-Vinylimidazol beschrieben. Aus EP 74191 sind Copolymere aus N-Vinylpyrrolidon,N-Vinylcaprolactam und Dimethylaminoethyl-methacrylat bekannt.

Bevorzugt werden für die erfindungsgemäßen Haarfestiger Homopolymere des N-Vinylcaprolactam verwendet.

Die erfindungsgemäßen Haarfestigungsmittel enthalten die Polymere A in einer Menge von 0,5 - 20, bevorzugt von 1 - 10 Gew.-%, bezogen auf das fertige Mittel.

Als Polymere B sind eine Vielzahl von in der Haarkosmetik verwendeten Polymeren einsetzbar.

Als Polymere B) kommen erfindungsgemäß von A) verschiedene weitere filmbildende Polymere, ausgewählt aus der Gruppe, bestehend aus Polyamiden, Polyurethanen und Homo- und Copolymeren von monoolefinisch ungesättigten Monomeren in Betracht. Filmbildende Polymere können erfindungsgemäß übliche Haarfestigungspolymere sein, aber auch Filmbildner für technische Beschichtungs- und Bindemittel.

Geeignete Homo- und Copolymere von monoolefinisch ungesättigten Monomeren sind vor allem Homo- und Copolymere von C₁-C₁₂-Alkylacrylaten und -methacrylaten, monoolefinisch ungesättigte C₃-C₅-Monocarbonsäuren und deren Vinylestern, Maleinsäure und deren Halbestern, Methylvinylether, Acrylamiden, Methacrylamiden, N-Alkylsubstituierten(meth)acrylamiden, N-Vinylpyrrolidon, N-Vinylimidazol sowie Styrol.

Besonders bevorzugte Polymere B) sind Acrylat-Polymere, wie Copolymere aus Acrylsäure/Ethylacrylat/N-tertiär-Butylacrylamid. Copolymere aus Methacrylsäure/tertiär-Butylacrylat/N-Vinylpyrrolidon, Copolymere auf Basis Acrylsäure und Vinylacetat, Copolymere aus Octylacrylamid/Acrylat/Butylaminoethylmethacrylat, Copolymere aus Octylacrylamid/Acrylat, Copolymere aus Ethylmethacrylat/Methacrylsäure/N-Vinylpyrrolidon oder Copolymere aus Acrylat und Hydroxyalkylacrylat. Solche Copolymere werden beispielsweise unter den Handelsnamen Amerhold®, Ultrahold® 8, Ultrahold Strong®, Luviflex® VBM, Luvimer® 100P, Luvimer® 36D, Luvimer® MAE30D, Acronal® 500D, Acudyne® 255, Stepanhold®, Lovocryl®, Versatyl®, Amphomer® vertrieben.

Bevorzugte Polymere B sind sulfonatgruppentragende Polyamide, bestehend aus:
- 20 bis 99 Mol.-% einer Monoaminocarbonsäure mit 2 bis 12 C-Atomen oder deren Lactam,
- 0,5 bis 40 Mol.-% eines Diamins mit 2 bis 18 C-Atomen,
- 0,5 bis 25 Mol.-% einer sulfonatgruppentragenden Dicarbonsäure mit 4 bis 12 C-Atomen, und
- 0 bis 35 Mol.-% einer weiteren Dicarbonsäure mit 2 bis 16 C-Atomen.

Weiterhin sind bevorzugte Polymere B) sulfonatgruppentragende Acrylate, bestehend aus:
- 50 bis 90 Gew.-% eines oder mehrerer Monomeren aus der Gruppen bestehend aus C₁-C₁₈-Alkylestern der Acrylsäure oder der Methacrylsäure und Vinylestern von gesättigten C₂-C₁₀-Monocarbonsäuren,
- 10 bis 25 Gew.-% eines sulfonsäuregruppenhaltigen Vinylmonomeren, und
- 0 bis 40 Gew.-% eines weiteren Monomeren aus der Gruppe bestehend aus Acrylsäure, Methacrylsäure, Acrylamid, Methacrylamid, C₃-C₈-N-Alkylacrylamid, N,N-Dimethylacrylamid.

Ebenfalls bevorzugte Polymere B) sind Copolymerisate in Form von Mikrodispersionen gemäß der DE-A 43 27 514, welche erhältlich sind durch radikalinitiierte oder durch Anwendung ionisierender Strahlung initiierte Copolymerisation von
- 40 bis 99 Gew.-% eines oder mehrerer wasserunlöslicher, monoethylenisch ungesättigter Monomeren,
- 1 bis 60 Gew.-% eines oder mehrerer wasserlöslicher, monoethylenisch ungesättigter Monomeren und
- 0 bis 30 Gew.-% eines oder mehrerer mehrfach ethylenisch ungesättigter Monomeren in wäßrigem Medium in Gegenwart von 2 bis 20 Gew.-%, bezogen auf die Gesamtmenge der Monomeren, oberflächenaktiver Verbindungen als Emulgatoren, mit einer mittleren Teilchengröße von 5 bis 37 nm, bestimmt durch Lichtstreuung in wäßrigem Medium.

Geeignete Polymere B) sind auch Homo- und Copolymere des N-Vinylpyrrolidons, wie sie beispielsweise unter dem Namen Luviskol® von der BASF Aktiengesellschaft vertrieben werden. Die Copolymere sind durch Polymerisation von N-Vinylpyrrolidon mit Vinylacetat und/oder Vinylpropionat in verschiedenen Gewichtsverhältnissen erhältlich. Beispiele für solche Polymere sind:

Luviskol® K17 Luviskol® K30 Luviskol® K60 Luviskol® K80, Luviskol® K90 (Polyvinylpyrrolidone mit entsprechendem K-Wert als Pulver oder als Lösung (wäßrig oder wäßrig/alkoholisch)).

Luviskol® VA = Vinylpyrrolidon/Vinylacetat-Copolymere, insbesondere Luviskol® VA 73, Luviskol® VA 64, Luviskol® VA 55, Luviskol® VA 37, Luviskol® VA 28.

Weitere geeignete Polymere B sind ternäre Polymere aus Vinylpyrrolidon, Vinylacetat und Vinylpropionat, beispielsweise Luviskol® VAP 343.

Ebenfalls geeignet sind Vinylacetat-Copolymere mit Crotonsäure beispielsweise die Produkte Luviset® CA, Luviset® CAP, National® 28-2930.

Es können auch quaternierte Polyquaternium-4, Polyquaternium-6, Polyquaternium-7, Polyquaternium-10, Polyquaternium-11 oder Polyquaternium 16 verwendet werden.

Weitere geeignete Polymere B) sind Copolymere aus Methylvinylether (MVE) und Maleaten, beispielsweise Copolymere aus MVE/Monoethylmaleat, MVE/Monoisopropylmaleat oder MVE/Monobutylmaleat, beispielsweise die Produkte Gantrez ES-225, Gantrez® ES-335, Gantrez® ES-425, Gantrez® ES-435, Gantrez® SP-215.

Auch Polyurethane, wie sie beispielsweise aus WO 94/03510 bekannt sind, können als Polymer B eingesetzt werden. Ebenfalls geeignet sind Natriumsalze der Polystyrolsulfonsäure wie sie unter dem Namen Flexan® 130 vertrieben werden.

Die Polymere A und B können, falls sie aus säuregruppenhaltigen Monomeren aufgebaut sind, in der Säureform oder teilweise oder komplett neutralisiert für die erfindungsgemäßen Haarfestigungsmittel verwendet werden. Zur Neutralisierung eignen sich Alkalihydroxide, Ammoniak, organische Amine, insbesondere Aminoalkohole, wobei 2-Amino-2-methyl-1-propanol ganz besonders bevorzugt wird.

Die Polymere A und B liegen in einem Lösungsmittel vor, wobei als Lösungsmittel bevorzugt Wasser oder Alkohole oder Mischungen aus Wasser und niederen Alkoholen eingesetzt werden. Der Lösungsmittelanteil im Haarfestigungsmittel beträgt üblicherweise zwischen 25 - 98 Gew.-%.

Das Zusammenmischen der Polymere A und B kann erfolgen, indem A und B als Pulver in einem Lösungsmittel aufgelöst werden, oder indem in eine Lösung eines Polymeren das jeweils andere Polymer als Feststoff oder ebenfalls als Lösung zugemischt wird. Polymer B kann auch als Dispersion vorliegen, wobei Polymer A als Pulver oder wäßrige bzw. wäßrig-alkoholische Lösung zugegeben werden kann.

Die erfindungsgemäßen Haarfestigungsmittel enthalten die Polymere B in einer Menge von 0,5 - 20,0 bevorzugt von 1 - 5 Gew.-%, bezogen auf das fertige Mittel.

Neben den Polymeren A und B sowie einem Lösungsmittel können die erfindungsgemäßen Haarfestigungsmittel je nach Verwendungszweck weitere übliche haarkosmetische Zusätze wie Parfümöle, Emulgatoren, Konservierungsmittel, Pflegestoffe wie Panthenol, Collagen, Vitamine, Eiweißhydrolysate, Stabilisatoren, pH-Wert-Regulatoren, Farbstoffe und weitere übliche Additive enthalten.

Soll das erfindungsgemäße Haarfestigungsmittel als Haarspray verwendet werden, so wird in der Regel ein Treibmittel zugesetzt. Übliche Treibmittel sind niedere Alkane, beispielsweise Propan oder Butan, Dimethylether, Stickstoff, Stickoxydul oder Kohlendioxid oder Gemische aus diesen Substanzen. Die erfindungsgemäßen Haarfestigungsmittel können auch halogenhaltige Kohlenwasserstoffe als Treibmittel enthalten.
Bei Verwendung in mechanischen Sprühvorrichtungen, beispielsweise Sprühpumpen, kann das Treibmittel entfallen.

Die erfindungsgemäßen Haarfestigungsmittel besitzen hervorragende anwendungstechnische Eigenschaften; sie bilden klare Filme und besitzen in wäßrig/alkoholischen Lösungen eine niedrige Lösungsviskosität, so daß sie auch höherkonzentriert noch gute Sprüheigenschaften besitzen. Überraschenderweise lassen sich auch wasserunslöliche filmbildende Polymere in Kombination mit Polyvinylcaprolactamen zu gut auswaschbaren Haarfestigungsmitteln verarbeiten.

Die in den nachstehenden Beispielen verwendeten Polyvinylcaprolactam-Lösungen waren 50 gew.-%ige Lösungen in Ethanol. Der K-Wert nach Fikentscher des Polymeren lag bei 40 (1 % in Ethanol).

### Beispiel 1

### Herstellung und Eigenschaften eines Haarfestigungsmittels

| | |
|---|---|
| 4,0 Gew.-% | Luvimer® 36D (36 gew.-%ige wäßrige Dispersion eines Copolymers aus tert-Butylacrylat, Ethylacrylat, Methacrylsäure) |
| 7,0 Gew.-% 89,0 Gew.-% +) K-Wert 40 | Polyvinylcaprolactam-Lösung dest. Wasser |

wurden bei Raumtemperatur gelöst und in eine Sprühpumpe gefüllt. Nach Besprühen zweier Modellköpfe ergab sich folgende Beurteilung des Mittels:

Die behandelten Haare wiesen bei gutem Glanz eine gute Festigkeit auf, ohne daß die Haare klebrig waren. Der Griff ist geschmeidig, die Kämmbarkeit und die Auswaschbarkeit gut.

### Beispiele 2 bis 9

Es wurden Aussehen, Eigenschaften behandelter Haarsträhnen und Klebrigkeit von Formulierungen der im folgenden genannten Zusammensetzungen ermittelt. Die Ergebnisse sind in der Tabelle aufgelistet.

Die Haarsträhnen wurden in den jeweiligen Formulierungen getränkt, auf Filterpapier leicht abgedrückt, getrocknet und auf ihre Steifigkeit untersucht.

Die Auswaschbarkeit wurde ermittelt, indem die wie oben behandelten Haarsträhnen in einer 37°C warmen, 10 Gew.-%igen wäßrigen Natriumlaurylethersulfat-Lösung 30 sec durch fünfmaliges Eintauchen gewaschen und asugedrückt wurden. Anschließend wurden die Haarsträhnen mit klarem Wasser gespült, der Auswaschvorgang wiederholt und die Haarsträhnen auf Filterpapier ausgedrückt und über Nacht getrocknet. Dann wurden die Haare auf Rückstände untersucht.

### Beurteilung der Klebrigkeit

Die Formulierungen wurden mit einer Rakel mit 120 µm Spaltbreite auf eine Glasplatte gestrichen. Der Naßfilm wurde bei Raumtemperatur getrocknet und anschließend im Klimaschrank bei 75 % relativer Luftfeuchtigkeit undf 20°C über Nacht gelagert. Zur Prüfung wurde ein Plastic-Carbonband mit einem runden Gummistempel (0̸ 40 mm, Shore A-Härte 60 ±5) mit 250 N für 10 sec auf dem Polymerfilm gepreßt. In dem Maß, in dem die Polymeroberfläche klebrig ist, bleibt die Druckfarbe des Carbon-Bandes auf dem Polymerfilm haften. Eine Klebrigkeit von 1 bedeutet, daß die Druckfarbe nur in äußerst geringem Maße auf dem Polymerfilm haften bleibt.

### Beispiel 2

| | |
|---|---|
| 5 Gew.-% | Polyvinylcaprolactam-Lösung |
| 10,0 Gew.-% | Polyurethan gemäß Beispiel 3, WO 94/03510 als 25 gew.-%ige wäßrige Mikrodispersion |
| 85,0 Gew.-% | aqua dest. |

### Beispiel 3

| | |
|---|---|
| 5 Gew.-% | Polyvinylcaprolactam-Lösung |
| 2,5 Gew.-% | Polyamid aus 20 Mol.-% ε-Caprolactam, 34 Mol.-% Hexamethylendiamin, 17 Mol.-% 5-Sulfoisophthalsäure-Natrium-Salz und 17 Mol.-% Isophthalsäure |
| 92,5 Gew.-% | aqua dest. |

### Beispiel 4

| | |
|---|---|
| 5 Gew.-% | Polyvinylcaprolactam |
| 10 Gew.-% | Acrylat-Mikrodispersion gemäß Beispiel 10, DE-A 43 27 514 (Festgehalt 20 Gew.-%) |
| 85 Gew.-% | aqua dest. |

### Beispiel 5

| | |
|---|---|
| 5,0 Gew.-% | Polyvinylcaprolactam-Lösung |
| 2,5 Gew.-% | Luviskol VA 64P, pulverförmiges Copolymer aus N-Vinylpyrrolidon/Vinylacetat |
| 52,5 Gew.-% | Ethanol abs. |
| 40,0 Gew.-% | aqua dest. |

### Beispiel 6

| | |
|---|---|
| 5 Gew.-% | Polyvinylcaprolactam-Lösung |
| 2,5 Gew.-% | Luvimer 100P, Pulver eines Copolymeren aus tertiärButylacrylat/Ethylacrylat/Methacrylsäure |
| 0,58 Gew.-% | 2-Amino-2-methyl-propanol (AMP) |
| 52,50 Gew.-% | Ethanol abs. |
| 39,42 Gew.-% | aqua dest. |

### Beispiel 7

| | |
|---|---|
| 5 Gew.-% | Polyvinylcaprolactam-Lösung |
| 2,5 Gew.-% | Luviset CAP, pulverförmiges Terpolymer aus Vinylacetat/Vinylpropionat/Crotonsäure |
| 0,19 Gew.-% | AMP |
| 52,50 Gew.-% | Ethanol abs. |
| 39,81 Gew.-% | aqua dest. |

### Beispiel 8

| | |
|---|---|
| 5 Gew.-% | Polyvinylcaprolactam-Lösung |
| 2,5 Gew.-% | Ultrahold 8, pulverförmiges Terpolymer aus N-tertiärButylacrylamid, Ethylacrylat, Acrylsäure |
| 0,23 Gew.-% | AMP |
| 52,50 Gew.-% | Ethanol abs. |
| 39,77 Gew.-% | aqua dest. |

### Beispiel 9

| | |
|---|---|
| 5 Gew.-% | Polyvinylcaprolactam-Lösung |
| 2,5 Gew.-% | Amphomer, Alkylacrylamid/Acrylat-Copolymer |
| 0,41 Gew.-% | AMP |
| 52,50 Gew.-% | Ethanol abs. |
| 39,59 Gew.-% | aqua dest. |

### Beispiel 10

| | |
|---|---|
| 6,8 Gew.-% | Polyvinylcaprolactam-Lösung |
| 3,4 Gew.-% 89,8 Gew.-% | Acronal 500D, 50 gew.-%ige wäßrige anionische Copolymerdispersion eines Acrylat/Vinylacetat-Copolymers aqua dest. |

**Tabelle**

| Bsp. Nr. | Aussehen | | Haarsträhne Steifeffekt | behandelt Auswaschbarkeit | Klebrigkeit aus 5 Gew.-%iger Lösung, bei 20°C, 80% rel.Feuchte |
|---|---|---|---|---|---|
| | Lösung | Film auf Glasplatte | | | |
| 2 | klar | klar | sehr gut | gut | 1 |
| 3 | klar | klar | sehr gut | gut | 1 |
| 4 | opaque | klar | sehr gut | gut | 1 |
| 5 | klar | klar | sehr gut | gut | 1 |
| 6 | klar | klar | sehr gut | gut | 1 |
| 7 | klar | klar | sehr gut | gut | 1 |
| 8 | klar | klar | sehr gut | gut | 1 |
| 9 | klar | fast klar | sehr gut | gut | 1 |
| 10 | milchig | fast klar | sehr gut | noch gut | 1 |

### Beispiel 11

| | |
|---|---|
| 5 Gew.-% | Polyvinylcaprolactam-Lösung |
| 2,5 Gew.-% | Luvimer 100P |
| 0,58 Gew.-% | AMP |
| 52,50 Gew.-% | Ethanol |
| 39,42 Gew.-% | (Mischung aus Propan/Butan im Verhältnis 50/50) |

### Beispiel 12

| | |
|---|---|
| 5 Gew.-% | Polyvinylcaprolactam-Lösung |
| 2,5 Gew.-% | Ultrahold 8 |
| 0,23 Gew.-% | AMP |
| 32,27 Gew.-% | Ethanol |
| 60 Gew.-% | (Mischung aus Propan/Butan/n-Pentan im Verhältnis 30/50/20) |

## Patentansprüche

1. Haarfestigungsmittel, enthaltend
A) 0,5 - 20 Gew.% eines Homopolymeren des N-Vinylcaprolactams oder eines anionischen oder nichtionischen Copolymeren aus mindestens 70 Gew.% N-Vinylcaprolactam (Polymer A), und
B) 0,5 - 20 Gew.% eines weiteren filmbildenden Polymeren (Polymer B), ausgewählt aus der Gruppe bestehend aus Polyamiden, Polyurethanen, Homo- und Copolymeren von monoolefinisch ungesättigten Monomeren.

2. Haarfestigungsmittel nach Anspruch 1, **dadurch gekennzeichnet, daß** als Polymer A ein Homopolymer aus N-Vinylcaprolactam eingesetzt wird.

3. Haarfestigungsmittel nach Anspruch 1 oder 2, **dadurch gekennzeichnet, daß** das Polymer A in einer Menge von 1-10 Gew.% eingesetzt wird.

4. Haarfestigungsmittel nach einem der Ansprüche 1 bis 3, **dadurch gekennzeichnet, daß** es einen Alkohol und niedere Alkane enthält.

## Claims

1. A hair setting lotion comprising
A) 0.5 - 20% by weight of a homopolymer of N-vinylcaprolactam or of an anionic or nonionic copolymer of at least 70% by weight of N-vinylcaprolactam (polymer A), and
B) 0.5 - 20% by weight of a further film-forming polymer (polymer B), selected from the group consisting of polyamides, polyurethanes, and homo- and copolymers of monoolefinically unsaturated monomers.

2. A hair setting lotion as claimed in claim 1, wherein the polymer A employed is a homopolymer of N-vinylcaprolactam.

3. A hair setting lotion as claimed in claim 1 or 2, wherein the polymer A is employed in an amount of 1-10% by weight.

4. A hair setting lotion as claimed in any of claims 1 to 3, containing an alcohol and lower alkanes.

## Revendications

1. Produit pour fixation capillaire, contenant
A) 0,5 - 20 % en poids d'un homopolymère de N-vinylcaprolactame ou d'un copolymère anionique ou non-ionique issu d'au moins 70 % en poids de N-vinylcaprolactame (polymère A) et
B) 0,5 - 20 % en poids d'un autre polymère filmogène (polymère B), choisi dans le groupe consistant en polyamides, polyuréthannes, homo- et copolymères de monomères à insaturation monooléfinique.

2. Produit pour fixation capillaire selon la revendication 1, **caractérisé par le fait qu'**on utilise comme polymère A un homopolymère de N-vinylcaprolactame.

3. Produit pour fixation capillaire selon la revendication 1 ou 2, **caractérisé par le fait que** le polymère A est mis en oeuvre en une quantité de 1 - 10 % en poids.

4. Produit pour fixation capillaire selon l'une des revendications 1 à 3, **caractérisé par le fait qu'**il contient un alcool et des alcanes inférieurs.
